# EUROPÄISCHE PATENTSCHRIFT

(11) **EP 2 427 229 B1**
(45) Veröffentlichungstag und Bekanntmachung des Hinweises auf die Patenterteilung: **04.03.2015**
(21) Anmeldenummer: 10717026.8
(22) Anmeldetag: 04.05.2010
(51) Int. Cl.: A61M 1/00, A61M 27/00, A61M 39/10, A61M 39/26

(54) **DRAINAGESCHLAUCHVORRICHTUNG UND KUPPLUNGSEINHEIT**
DRAINAGE TUBE APPARATUS AND COUPLING UNIT
DISPOSITIF À TUYAU SOUPLE DE DRAINAGE ET UNITÉ D'ACCOUPLEMENT

(30) Priorität: 08.05.2009 CH 726092009
(43) Veröffentlichungstag der Anmeldung: 14.03.2012
(73) Patentinhaber: Medela Holding AG, 6340 Baar (CH)
(72) Erfinder: HOFMANN, Adrian, CH-6005 Luzern (CH); HENZEN, Ignaz, CH-6331 Hünenberg (CH); ZANETTI, Daniel, CH-6330 Cham (CH)
(74) Vertreter: Clerc, Natalia
(86) Internationale Anmeldenummer: PCT/CH2010/000114
(87) Internationale Veröffentlichungsnummer: WO 2010/127461

(56) Entgegenhaltungen:
- EP-A2- 1 920 791
- US-A- 3 851 650
- US-A- 4 541 457

## Beschreibung

### TECHNISCHES GEBIET

Die vorliegende Erfindung betrifft eine Drainageschlauchvorrichtung sowie eine Kupplungseinheit zur Verwendung mit einer derartigen Drainageschlauchvorrichtung.

### STAND DER TECHNIK

Drainagesysteme zum Absaugen von Körperflüssigkeiten, beispielsweise für die Thoraxdrainage, für die Fettabsaugung, für die Blut- oder Sekretabsaugung während Operationen und für die Wunddrainage, sind bekannt.

Hierfür wird beispielsweise ein Katheter in eine Körperkavität oder ein Saugkopf wird zu einer Wunde geführt. Der Katheter oder der Saugkopf ist mit einer Saugleitung verbunden, welche in einen Sekretsammelbehälter führt. Mittels eines Zentralvakuumsystems oder einer Saugpumpe wird in der Saugleitung ein Unterdruck erzeugt, so dass die Körperflüssigkeit durch die Saugleitung in den Sammelbehälter gesogen wird. Es ist ferner bekannt, zusätzlich zur Saugleitung noch eine Serviceleitung zu verwenden, welche beispielsweise eine Entlüftung der Saugleitung ermöglicht.

So offenbart WO 94/20152 eine Drainagevorrichtung zum Absaugen von Fluiden aus einer Körperhöhle, insbesondere der Pleurahöhle. Die Vorrichtung weist eine Drainageleitung zum Absaugen der Fluide und eine Vakuumpumpe zum Erzeugen eines Unterdrucks in der Körperhöhle auf. Über eine Zusatzleitung kann ein Gas in die Körperhöhle eingeführt werden. Diese Zusatzleitung steht am patientenseitigen Ende in Fluidverbindung mit der Drainageleitung. Vorzugsweise sind Zusatzleitung und Drainageleitung in einem doppellumigen Schlauch angeordnet. Am patientenfernen Ende der Zusatzleitung ist ein Druckmessgerät angeordnet und es ist vorgeschlagen, die Druckdifferenz zwischen den Strömen in der Zusatzleitung und der Drainageleitung zu bestimmen.

US 3 851 650 zeigt einen Drainageschlauch mit einem Anschlussteil zur Verbindung mit einem Katheter. Der Drainageschlauch ist doppellumig ausgeführt und verfügt über eine Drainageleitung zur Absaugung der Körperflüssigkeit eines Patienten und über eine Zusatzleitung, welche der Luftzufuhr dient. Um zu verhindern, dass abgesaugte Körperflüssigkeit in die Zusatzleitung gelangen kann, ist am patientenseitigen Ende der Zusatzleitung ein Einwegventil angeordnet, d.h. das Ventil öffnet in eine und schliesst in der entgegen gesetzten Richtung.

EP 0 599 472 offenbart ebenfalls die Verwendung eines doppellumigen Schlauchs in einem medizinisch-chirurgischen Saugsystem.

Diese Schläuche benötigen Kupplungseinheiten, sei es beispielsweise, um sie mit dem Katheter für die Absaugung oder einem Saugkopf für einen Wundverband zu verbinden oder um die Gesamtlänge des Drainageschlauches zu verlängern. Damit beim Lösen der Kupplungseinheit kein Körpersekret austritt, wird der Drainageschlauch üblicherweise zuerst mit einer Klemme verschlossen. Dies erschwert jedoch die Handhabung und benötigt mehr Zeit beim Wechseln der Drainagevorrichtung. Zudem wird dabei zeitweise vergessen, die Klemme zu schliessen. Auch bei neu erstellter Verbindung wird zeitweise vergessen, die Klemme wieder zu öffnen, so dass die Drainage nicht fortgeführt wird. Selbst wenn ein Überwachungssystem vorhanden ist, dauert es trotzdem eine geraume Zeit, bis eine Fehlermeldung erfolgt. Des Weiteren sollte die Kupplungseinheit eine Belüftung der Saugleitung mittels einer Zusatzleitung und/oder eine Druckmessung in der Saugleitung nicht beeinträchtigen.

Zumindest aus artfremden Bereichen sind Kupplungseinheiten bekannt, welche integrierte Ventile aufweisen und welche so ein Nachtropfen eines entkoppelten Schlauchs verhindern.

So beschreiben WO 2007/014281 und EP 1 840 443 Kupplungseinheiten zur fluiddichten Verbindung von zwei Schläuchen, wobei in einem Teil der Kupplungseinheit jeweils ein Ventil angeordnet ist.

US 4 541 457 offenbart eine Kupplungseinheit für eine CO₂-Leitung. Die Einheit umfasst ein männliches und ein weibliches Kupplungsteil. Im weiblichen Kupplungsteil ist ein Ventil angeordnet, welches eine Strömung in zwei entgegen gesetzte Richtungen ermöglicht bzw. verhindert, im männlichen Kupplungsteil ist ein Rückschlagventil vorhanden.

US 4 509 554 zeigt eine Kupplungseinheit für Hoch- und Niedrigdruckanwendungen mit einem männlichen und einem weiblichen Kupplungsteil. Beide Kupplungsteile sind je mit einem inneren und einem äusseren Federventil ausgestattet.

### DARSTELLUNG DER ERFINDUNG

Es ist deshalb eine Aufgabe der Erfindung, eine verbesserte Drainageschlauchvorrichtung und eine Kupplungseinheit zu schaffen, welche insbesondere eine Druckmessung ermöglichen.

Diese Aufgabe lösen eine Drainageschlauchvorrichtung und eine Kupplungseinheit mit den Merkmalen des Patentanspruchs 1 bzw. 14.

Die erfindungsgemässe Drainageschlauchvorrichtung zum Absaugen von Flüssigkeiten aus einem menschlichen oder tierischen Körper weist auf:
eine Kupplungseinheit mit einem ersten und einem zweiten Kupplungsteil, welche miteinander fluiddicht verbindbar sind,
ein erstes Ventil, welches im ersten Kupplungsteil angeordnet ist und
einen Drainageschlauch mit einem patientenseitigen Ende, welches im zweiten Kupplungsteil gehalten ist.

Der Drainageschlauch ist mehrlumig ausgebildet, und er weist eine Drainageleitung und mindestens eine Zusatzleitung auf. Diese Drainageleitung und mindestens eine der genannten Zusatzleitungen stehen an ihrem patientenseitigen Ende und innerhalb des zweiten Kupplungsteils miteinander in fluid-kommunizierender Verbindung. Die Drainageschlauchvorrichtung weist ferner ein zweites Ventil auf, welches im zweiten Kupplungsteil angeordnet ist und welches die Drainageleitung öffnet und verschliesst.

Die erfindungsgemässe Kupplungseinheit zur Verwendung in einer Drainageschlauchvorrichtung weist ein erstes und ein zweites Kupplungsteil auf, welche miteinander fluiddicht verbindbar sind. Es weist ferner ein erstes Ventil auf, welches im ersten Kupplungsteil angeordnet ist. Das zweite Kupplungsteil weist eine Aufnahme zur Befestigung eines patientenseitigen Endes eines mehrlumigen Drainageschlauchs auf, welcher eine Drainageleitung und mindestens eine Zusatzleitung aufweist, wobei die Aufnahme eine fluidkommunizierende Verbindung zwischen dieser Drainageleitung und mindestens einer der genannten Zusatzleitungen an ihrem patientenseitigen Ende innerhalb des zweiten Kupplungsteils ermöglicht. Die Kupplungseinheit weist ferner ein zweites Ventil auf, welches im zweiten Kupplungsteil angeordnet ist und welches die Drainageleitung öffnet und verschliesst.

Diese Drainageschlauchvorrichtung und diese Kupplungseinheit ermöglichen eine einfache Verbindung und Trennung des Drainageschlauchs mit weiteren Komponenten, insbesondere mit einem weiteren Drainageschlauch, einem Katheter oder einem Saugkopf, ohne dass Klemmen benötigt werden. Dank der Ventile wird ein Nachtropfen verhindert.

Das erste Ventil ist vorzugsweise im getrennten Zustand der Kupplungsteile stets geschlossen und das zweite Ventil ist vorzugsweise bei Fehlen eines Unterdrucks in der Drainageleitung stets geschlossen.

Sind die zwei Kupplungsteile lösbar über eine Schnappverbindung miteinander verbunden, so ist eine einfache Verbindung und Trennung der zwei Teile gewährleistet. Die zwei Kupplungsteile können unmittelbar miteinander verbunden sein. Sie können jedoch auch über ein Überbrückungsteil mittelbar miteinander verbunden werden. Dieses Überbrückungsteil weist vorzugsweise eine erste und eine zweite Endkomponente auf, wobei die erste Endkomponente mit dem ersten Kupplungsteil und die zweite Endkomponente mit dem zweiten Kupplungsteil fluiddicht verbindbar sind. Dabei ist die erste Endkomponente vorzugsweise gleich ausgebildet wie das zweite Kupplungsteil und die zweite Endkomponente gleich wie das erste Kupplungsteil. Vorzugsweise weist das Überbrückungsteil jedoch keine eigenen Ventile auf.

Die Verwendung eines mehrlumigen Schlauchs versteift die Drainageleitung und verhindert ein Abknicken der Leitung. Vorteilhaft ist die Verwendung von mehreren, vorzugsweise von genau drei Zusatzleitungen, welche peripher und gleichmässig verteilt um die zentral verlaufende Drainageleitung angeordnet sind.

Vorzugsweise ist im zweiten Kupplungsteil am patientenseitigen Ende des Drainageschlauchs ein Hohlraum vorhanden, welcher die kommunizierende Verbindung zwischen der Drainageleitung und der mindestens einen Zusatzleitung ermöglicht. Dieser Hohlraum erlaubt eine Druckmessung, insbesondere eine Druckdifferenzmessung zwischen der mindestens einen Zusatzleitung und der Drainageleitung. Dadurch ist eine patientennahe Druckmessung möglich, ohne dass die Sensoren im Kupplungsteil angebracht sein müssen.

Vorzugsweise ist das erste Ventil ein Ventil, welches eine Strömung in beiden entgegen gesetzten Richtungen zulässt oder verhindert. In einer bevorzugten Ausfuhrungsform weist das erste Ventil einen elastisch deformierbaren Kolben auf, welcher im ersten Kupplungsteil gehalten ist und dessen Deformierung bei Verbindung des ersten Kupplungsteils mit dem zweiten Kupplungsteil respektive, falls vorhanden, mit dem Überbrückungsteil erfolgt. Andere Arten derartiger Ventile lassen sich jedoch ebenfalls einsetzen, beispielsweise Federventile.

Das zweite Ventil kann ein Einventil sein, und eine Strömung nur in einer Richtung zulassen, oder es kann die Strömung in beiden entgegen gesetzten Richtungen zulassen. Vorzugsweise verschliesst es nur die Drainageleitung, nicht aber die Zusatzleitung. Vorzugsweise ist es so angeordnet, dass im geschlossenen Zustand des zweiten Ventils am patientenseitigen, d.h. dem kupplungsteilseitigen, Ende des Schlauchs keine kommunizierende Verbindung zwischen der Drainageleitung und der mindestens einen Zusatzleitung besteht.

Ist das zweite Ventil als Entenschnabelventil ausgebildet, benötigt es relativ wenig Platz und die Kupplungseinheit kann relativ kostengünstig hergestellt werden. Eine speziell einfache und kostengünstige Bauweise wird ermöglicht, wenn das zweite Ventil auf das patientenseitige Ende des Drainageschlauchs aufgesteckt ist, bzw. mindestens teilweise in die Drainageleitung hineinragt. Auf diese Art kann es gemeinsam mit dem Drainageschlauch in das Kupplungsteil eingeführt werden.

Üblicherweise wird der Drainageschlauch in das Kupplungsteil eingeklebt oder mit ihm verschweisst. Vorzugsweise wird das zweite Ventil zwischen einem Anschlag des zweiten Kupplungsteils und dem patientenseitigen Ende des Drainageschlauchs in einem Presssitz gehalten.

Die erfindungsgemässe Kupplungseinheit weist wenige Bauteile auf und ist deshalb einfach und kostengünstig herstellbar. Des Weiteren gewährleistet es eine relativ hohe Funktionsfähigkeit im Gebrauch.

Weitere vorteilhafte Ausfuhrungsbeispiele sind in den abhängigen Ansprüchen beschrieben.

### KURZE BESCHREIBUNG DER ZEICHNUNGEN

Eine bevorzugte Ausführungsform der Erfindung wird im Folgenden anhand der Zeichnungen beschrieben, die lediglich zur Erläuterung dienen und nicht einschränkend auszulegen sind. In den Zeichnungen zeigen:
- Fig. 1: eine Explosionsdarstellung der erfindungsgemässen Drainageschlauchvorrichtung;
- Figur 2: die Drainageschlauchvorrichtung gemäss Figur 1 mit entkoppelter Kupplungseinheit;
- Figur 3: einen Längsschnitt durch ein erstes Kupplungsteil der Kupplungseinheit gemäss Figur 2;
- Figur 4: einen Längschnitt durch eine Drainageschlauchvorrichtung gemäss Figur 1 im zusammengesteckten Zustand der Kupplungseinheit;
- Figur 5: einen Längschnitt der Drainageschlauchvorrichtung gemäss Figur 4 in einer Ebene senkrecht zu dieser Figur;
- Figur 6: eine schematische Darstellung der erfindungsgemässen Drainageschlauchvorrichtung mit einer Drainageeinheit;
- Figur 7: eine schematische Darstellung der erfindungsgemässen Kupplungseinheit im entkoppelten Zustand;
- Figur 8: die Kupplungseinheit gemäss Figur 7 im zusammengesteckten Zustand;
- Figur 9: die Kupplungseinheit gemäss Figur 7 im Drainagebetrieb mit angelegtem konstantem Unterdruck;
- Figur 10: die Kupplungseinheit gemäss Figur 7 im Drainagebetrieb bei intermittierendem Unterdruck;
- Figur 11: die Kupplungseinheit gemäss Figur 7 bei Spülung der Leitung;
- Figur 12: die Kupplungseinheit gemäss Figur 7 bei einer Druckmessung und
- Figur 13: eine erfindungsgemässe Kupplungseinheit mit einem Überbrückungsteil.

### BESCHREIBUNG BEVORZUGTER AUSFÜHRUNGSFORMEN

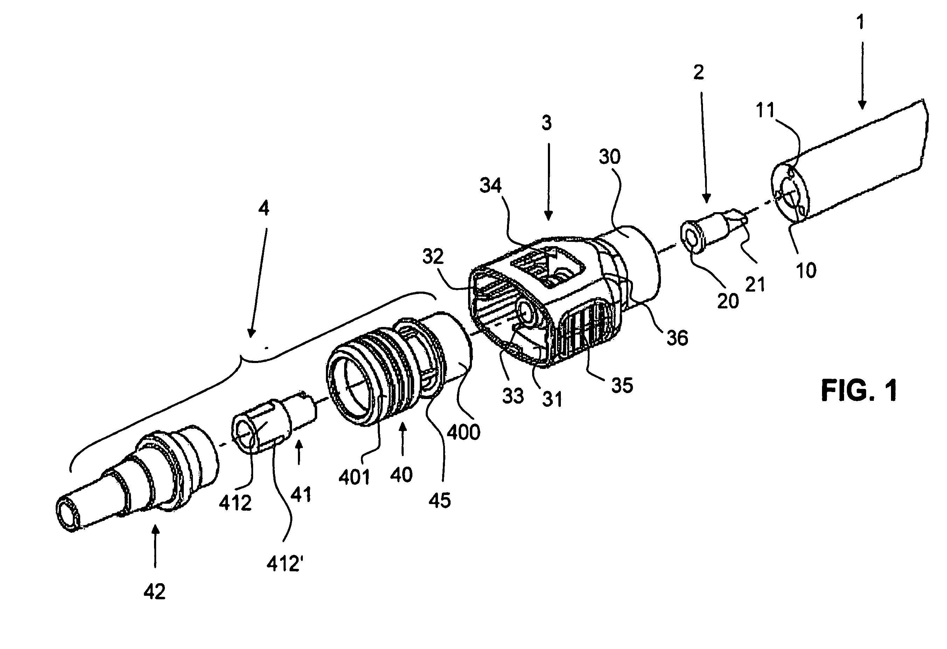

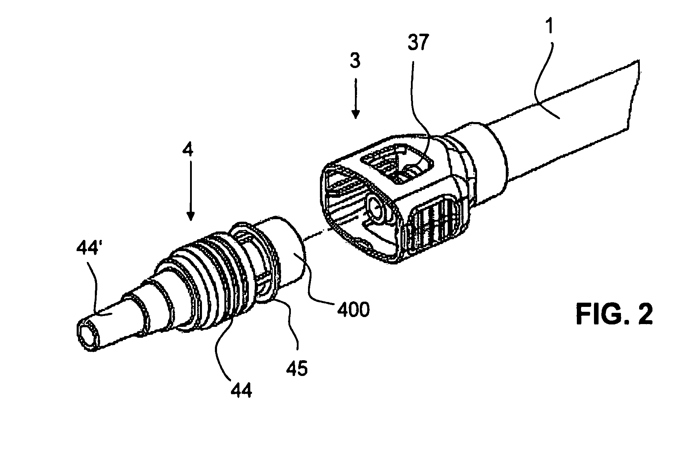

In den Figuren 1 und 2 ist eine erfindungsgemässe Drainageschlauchvorrichtung dargestellt. Sie weist ein erstes Kupplungsteil 4 mit einem ersten Ventil 41, einen Drainageschlauch 1, ein zweites Ventil 2 sowie ein zweites Kupplungsteil 3 auf.

Der Drainageschlauch 1 ist ein mehrlumiger Schlauch, vorzugsweise aus PVC. Er weist mindestens zwei Leitungen auf, eine Drainageleitung 10 und eine Zusatzleitung 11. Vorzugsweise verläuft die Drainageleitung 10 zentral und die Zusatzleitung im peripheren Bereich. Der Durchmesser der Zusatzleitung 11 ist vorzugsweise um ein Vielfaches kleiner als derjenige der Drainageleitung 10. Die Zusatzleitung 11 und die Drainageleitung 10 verlaufen bevorzugt vollständig getrennt voneinander und weisen keine Verbindung zueinander auf.

In der dargestellten Ausfuhrungsform sind drei Zusatzleitungen 11 vorhanden, welche gleichmässig über den Umfang verteilt um die Drainageleitung angeordnet sind. Die drei Zusatzleitungen 11 weisen vorzugsweise alle dieselbe Querschnittsfläche auf. Sie können jedoch auch unterschiedlich grosse Lumen aufweisen.

Dieser Drainageschlauch 1 lässt sich in bekannter Weise, beispielsweise im Extrusionsverfahren, herstellen.

Das hier dargestellte zweite Ventil 2 ist ein Einwegventil. Es könnte jedoch auch ein sich gegenüber der Strömung in beiden entgegen gesetzten Richtungen verschliessendes und öffnendes Ventil sein. Hier wird ein Entenschnabelventil (duck bill valve) verwendet. Es besteht vorzugsweise aus Silikon. Es weist einen umlaufenden, radial vorstehenden und ebenen Flansch 20 sowie einen Schnabel 21 auf, welcher bei entsprechendem äusseren einseitigen Druck öffnet bzw. bei Fehlen dieses Drucks schliesst. Der Schnabel 21 ist zur Drainageleitung 10 hin gerichtet.

Der Drainageschlauch 1 ist mit dem zweiten Kupplungsteil 3 lösbar, vorzugsweise fest verbunden. Vorzugsweise ist er mit seinem patientenseitigen Ende in dieses Kupplungsteil 3 eingeschweisst oder mit ihm verklebt. Das nicht dargestellte zweite Ende des Drainageschlauchs 1 führt zu einer Saugeinheit, beispielsweise einer Vakuumpumpe oder einem Zentralvakuum.

Das zweite Kupplungsteil 3 und das erste Kupplungsteil 4 sind vorzugsweise aus Kunststoff gefertigt. Das zweite Kupplungsteil 3 besteht bevorzugt aus PVC oder ABS. Das erste Kupplungsteil 4 besteht bevorzugt aus Hart-PVC, PC oder ABS. Sie sind vorzugsweise einstückig ausgebildet.

Das zweite Kupplungsteil 3 weist einen hohlzylinderförmigen Anschlussstutzen 30 zur Aufnahme des Ventils 2 und des Drainageschlauchs 1 auf. Der Anschlussstutzen 30 weist vorzugsweise einen runden Querschnitt auf. An diesem Anschlussstutzen 30 ist ein hohler Grundkörper 36 angeformt, welcher bevorzugt einen annähernd rechteckförmigen Querschnitt mit konvex gewölbten breiten Seitenflächen aufweist. Er weist an seinen zwei einander gegenüberliegenden schmalen Seiten je einen Druckbereich auf, welcher von Hand leicht eindrückbar ist, um den Grundkörper 36 in seiner Form zu verformen. Die Verformbarkeit wird durch die zwei Fenster 34, welche in den breiten Seiten des Grundkörpers 36 angeordnet sind, erleichtert. Dadurch lässt sich das nachfolgend beschriebene erste Kupplungsteil 4 auf einfache Art und Weise vom zweiten Kupplungsteil 3 entkoppeln. Seitenrippen 32, welche nach innen in den Grundkörper 36 vorstehend an den schmalen Seiten angeordnet sind, dienen als Anschläge beim Zusammendrücken des Grundkörpers 36.

Der Grundkörper 36 umschliesst eine Einführöffnung 31, in welcher ein Einsteckstutzen 33 beabstandet zu den seitlichen Wänden des Grundkörpers 36 in diesen hineinragt und das Gegenstück zum Anschlussstutzen 30 bildet. In diese Einführöffnung 31 und den Einsteckstutzen 33 umgreifend, lässt sich das erste Kupplungsteil 4 einstecken. Es können Rippen 37 oder Dichtwülste vorhanden sein. Diese sind den äusseren Umfang des Einsteckstutzens 33 umlaufend auf dem Einsteckstutzen 33 angeordnet und halten dabei das erste Kupplungsteil 4 in lösbarer Verbindung mit dem zweiten Kupplungsteil 3.

Das erste Kupplungsteil 4 kann einstückig oder mehrstückig ausgebildet sein. Im Falle einer Mehrstückigkeit sind die einzelnen Teile jedoch vorzugsweise fest und nicht zerstörungsfrei lösbar miteinander verbunden. Sie sind vorzugsweise miteinander verklebt oder verschweisst oder mittels Rückhalterippen schwer lösbar miteinander verbunden.

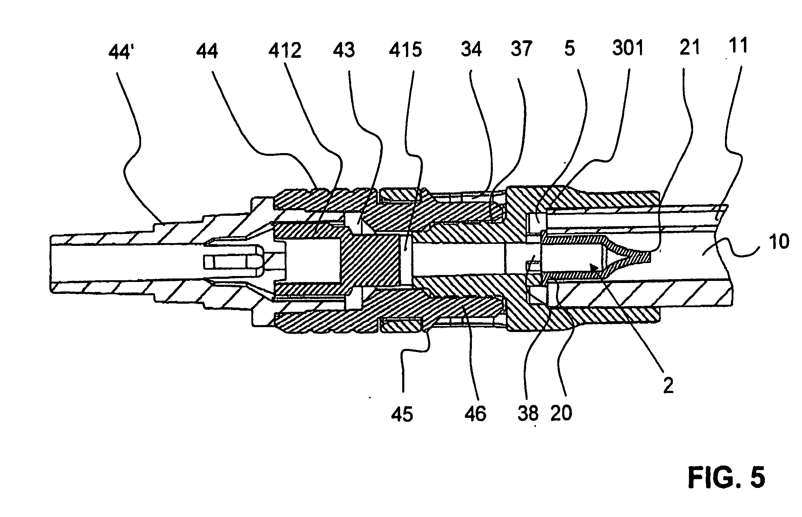

Das erste Kupplungsteil 4 weist einen hohlzylinderförmigen Grundkörper 40 auf, welcher zum zweiten Kupplungsteil 3 hin einen hohlzylinderförmigen Anschlussstutzen 400 aufweist. Dieser lässt sich über den Einsteckstutzen 33 schieben. Ein Flansch 45, welcher diesem Stutzen 400 radial vorsteht und zwischen Grundkörper 40 und Stutzen 400 verläuft, hintergreift im zusammen geschobenen Zustand das Fenster 34 und dient so als Schnappelement der Schnappverbindung zwischen den zwei Kupplungsteilen 3, 4. Dies ist in Figur 5 erkennbar.

Wie weiter in den Figuren 1 und 2 erkennbar ist, ist im ersten Kupplungsteil 4 ein erstes Ventil 41 angeordnet. Dieses wird später im Detail beschrieben. Das erste Kupplungsteil 4 weist ferner einen Schlauchanschluss 42 auf, um die Drainageschlauchvorrichtung an ein geeignetes Verbindungselement für die gewünschte Drainageanwendung anzuschliessen. Dies kann ein Katheter, ein weiterer Schlauch, ein Saugkörper oder ein anderes geeignetes Verbindungselement sein.

Der Schlauchanschluss 42 weist vorzugsweise eine sich zu seinem freien Ende verjüngende Struktur auf, um den Anschluss zu erleichtern. Die Struktur kann an ihrem äusseren Umfang mit Rippen, Stufen 44', einer Tannenbaumstruktur oder anderen geeigneten Mitteln versehen sein. Vorzugsweise weist der Anschluss 42 oder wie hier dargestellt der Grundkörper 40 ein Aussengewinde 44 auf, um eine weitere Anschlussmöglichkeit zu bieten.

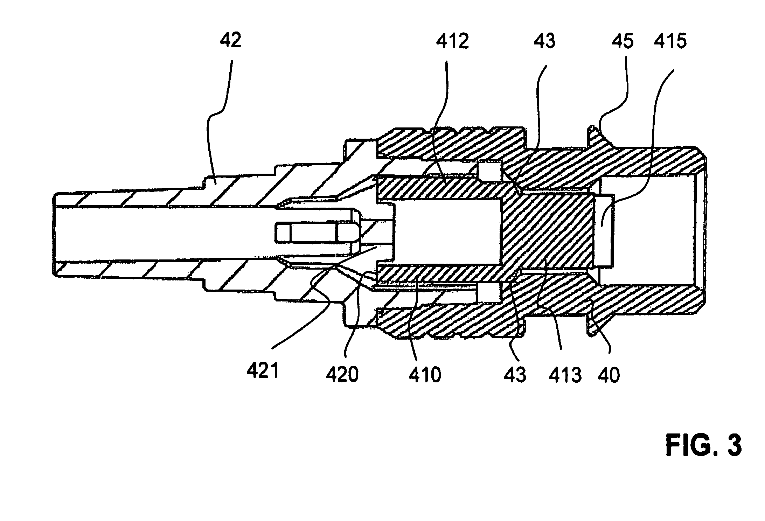

In Figur 3 ist erkennbar, wie das erste Ventil 41 zwischen Grundkörper 40 und Schlauchanschluss 42 gehalten ist. Das erste Ventil 41 weist einen ersten hohlzylinderförmigen Ventilkörper 410 und einen daran angeformten zweiten Ventilkörper 413 auf. Der zweite Ventilkörper 413 ist ebenfalls ein Kreiszylinder. Er ist jedoch massiv, d.h. ausgefüllt, ausgebildet. Am äusseren Umfang des ersten Ventilkörpers 410 sind vorzugsweise Rippen 412 bzw. Ausnehmungen 412' vorhanden, wie dies in den Figuren 1 und 3 erkennbar ist. Der zweite Ventilkörper 413 ist an seinem freien Ende plan ausgebildet, wobei in dieser Ebene ein Schlitz 415 verläuft, welcher sich vorzugsweise diametral über die gesamte Breite des Ventilkörpers erstreckt. Dieses erste Ventil ist vorzugsweise aus Silikon gefertigt.

Der Schlauchanschluss 42 weist an seinem dem Grundkörper 40 zugewandten Ende eine Stützstruktur 421 auf, welche einen umlaufenden Vorsprung 420 bildet. Auf diesem Vorsprung 420 liegt das zweite Ventil 41 mit einem planen Ende seines ersten Ventilkörpers 410 auf bzw. er steht an diesem Anschlag 420 an.

Wie in Figur 3 erkennbar ist, schliesst das erste Ventil 41 den im ersten Kupplungsteil 4 verlaufenden Durchgangskanal fluiddicht ab. Der umlaufende Dichtbereich ist in der Figur 3 mit dem Bezugszeichen 43 versehen.

Wie in Figur 2 dargestellt ist, lässt sich dieses zusammengesetzte erste Kupplungsteil 4 nun mit dem zweiten Kupplungsteil 3 und dem damit verbundenen Drainageschlauch 1 in einer lösbaren Steckverbindung verbinden.

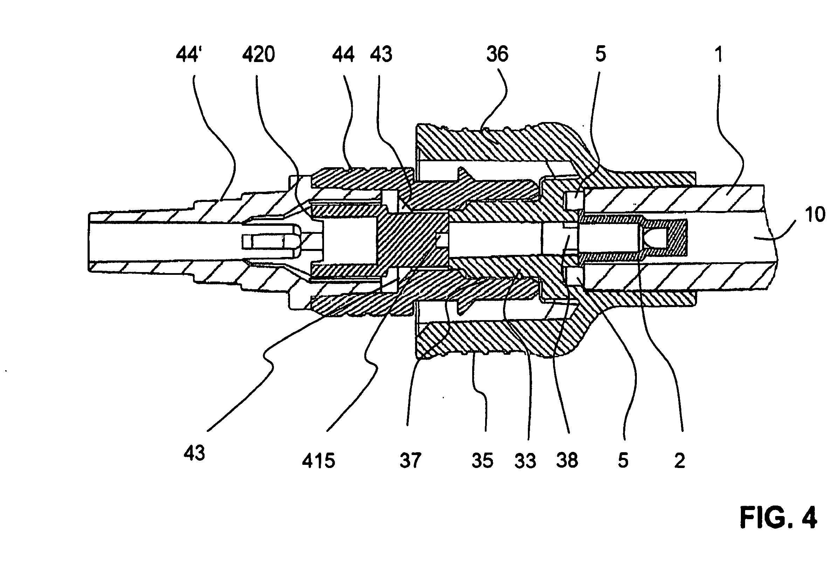

Dieser zusammengesteckte Zustand ist in den Figuren 4 und 5 dargestellt. Ebenfalls in diesen Figuren ist erkennbar, wie der Drainageschlauch 1 mit dem zweiten Kupplungsteil 3 verbunden ist. Dies sei hier nun zuerst erläutert:

Das zweite Kupplungsteil 3 weist im Innern des Anschlussstutzens einen umlaufenden Vorsprung 301 auf, welcher als Anschlag für das plane Ende des Drainageschlauchs 1 dient. Der Absatz ist so schmal ausgebildet, dass dabei nicht nur die Drainageleitung 10, sondern auch die Zusatzleitungen 11 in diesen gemeinsamen Hohlraum 5 münden. Vorzugsweise ist der Drainageschlauch 1 in dieser Position mit dem Anschlussstutzen verschweisst oder an ihn angeklebt.

Das zweite Ventil 2 ist mit seinem Schnabel 21 in die Drainageleitung 10 eingesteckt, so dass er sich zur Drainageleitung 10 hin öffnet. Er liegt dabei mit seinem Flansch 20 auf dem planen Ende des Drainageschlauchs 1 auf. Dabei überdeckt es die Mündungsöffnungen der Zusatzleitungen 11 nicht. Der Flansch 20 steht auf seiner gegenüberliegenden Seite an einer Krone, einem Schlitzring bzw. einem Gitter 38 des zweiten Kupplungsteils 3 an, so dass er im Presssitz zwischen Drainageschlauch 1 und diesem Gitter 38 gehalten ist. Der Gitter 38 befindet sich im Hohlraum 5, wobei er eine Fluidkommunikation zwischen den Zuleitungen 11 und der Drainageleitung 10 nicht behindert.

Wie nun in den Figuren 4 und 5 erkennbar ist, ist der Einsteckstutzen 33 in die Aufnahme des Anschlussstutzens 400 eingeführt. Er stösst im zusammengesteckten Zustand der Kupplungseinheit in Richtung des Vorsprungs 420 des ersten Kupplungsteils 4. Dadurch wird das erste Ventil 41, insbesondere der erste Ventilkörper 410 zusammengedrückt und verformt. Der Dichtbereich 43 wird freigegeben und es entsteht dank dem Schlitz 415 ein durchgehender Kanal zwischen dem freien Ende des ersten Kupplungsteils 4 und freien Ende des zweiten Kupplungsteils 3 bzw. dem Drainageschlauch 1. Dieser Kanal ist in der Figur 5 am besten erkennbar.

Das erste Ventil 41 wirkt somit als ein in beiden Richtungen öffnendes und verschliessendes Ventil, solange die zwei Kupplungsteile 3, 4 miteinander verbunden sind. Sobald das zweite Kupplungsteil 3 wieder entfernt wird, nimmt das elastisch ausgebildete zweite Ventil 41 wieder seine ursprüngliche Form an und schliesst den Dichtbereich 43. Das erste Kupplungsteil 4 ist somit nur im zusammen gesteckten Zustand der Kupplungseinheit offen.

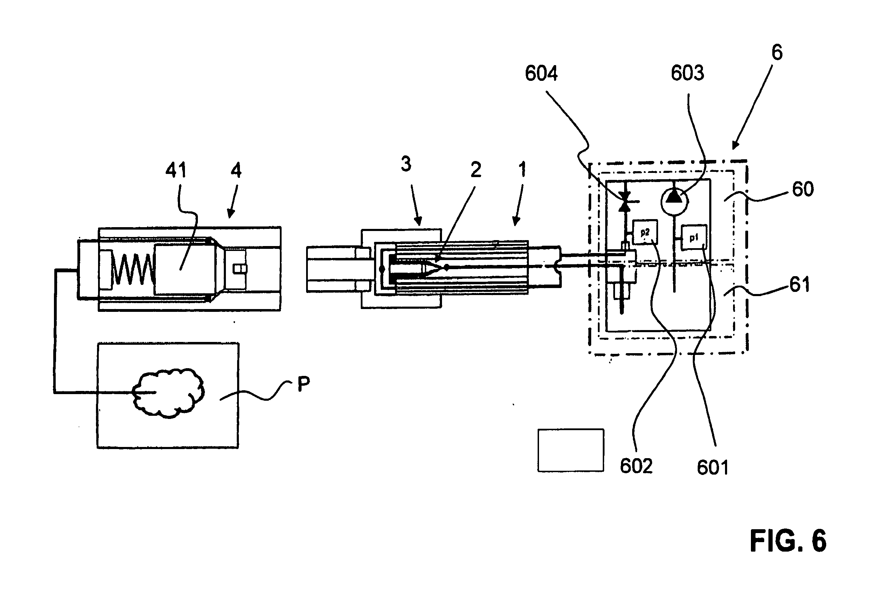

Anstelle des elastisch verformbaren ersten Ventils 41 lässt sich auch ein anderes selbsttätig schliessendes Ventil verwenden, beispielsweise ein federbelastetes Ventil. Dies ist schematisch in den Figuren 6 bis 12 dargestellt.

Das zweite Ventil 2 ist bei Fehlen eines Unterdrucks in der Drainageleitung 10 stets geschlossen.

Die Kupplung zwischen den zwei Kupplungsteilen 3, 4 wird gelöst, indem auf die seitlichen Druckbereiche 35 gedrückt wird, bis der Flansch 45 des ersten Kupplungsteils 4 vom Grundkörper 36 des zweiten Kupplungsteils 3 frei gegeben wird. Dadurch wird eine einfach bedienbare und doch sichere und fluiddichte Schnellkupplung geschaffen.

In Figur 6 ist die Drainageschlauchvorrichtung in einer Drainageanwendung dargestellt. P bezeichnet den Patienten. Der Drainageschlauch 1, genauer die Drainageleitung 10, ist mit einer Drainageeinheit 6 verbunden und führt in einen Drainagebehälter 61. Vom Drainagebehälter 61 führt eine Leitung zu einem Pumpaggregat 603 einer Vakuumpumpe 60. Auch die Zusatzleitung 11 führt in die Drainageeinheit, wobei sie in die Vakuumpumpe 60 mündet.

Ein in der Saugpumpe angeordnetes Rückschlagventil 604 ermöglicht in seiner offenen Stellung einen Spülbetrieb. Das heisst, Luft kann über die Zusatzleitung 11 in den Hohlraum 5 eingeführt werden und die Drainageleitung 10 kann mit Luft gespült werden.

In der Saugpumpe 60 bzw. im Bereich zwischen Saugpumpe und Drainageschlauch 1 ist mindestens ein Drucksensor vorhanden, um den Druck im Hohlraum 5 zu bestimmen. Hier sind zwei Drucksensoren 601, 602 vorhanden, so dass eine Druckdifferenz zwischen Drainageleitung 10 und Zusatzleitung 11 gemessen werden kann. Als Drucksensoren lassen sich handelsübliche bekannte Sensoren einsetzen.

Die Zusatzleitungen 11 dienen somit einerseits der Messung des Drucks im Hohlraum bzw. des Differenzdrucks zur Drainageleitung 10 wie auch zur Entlüftung und Spülung der Drainageleitung 10.

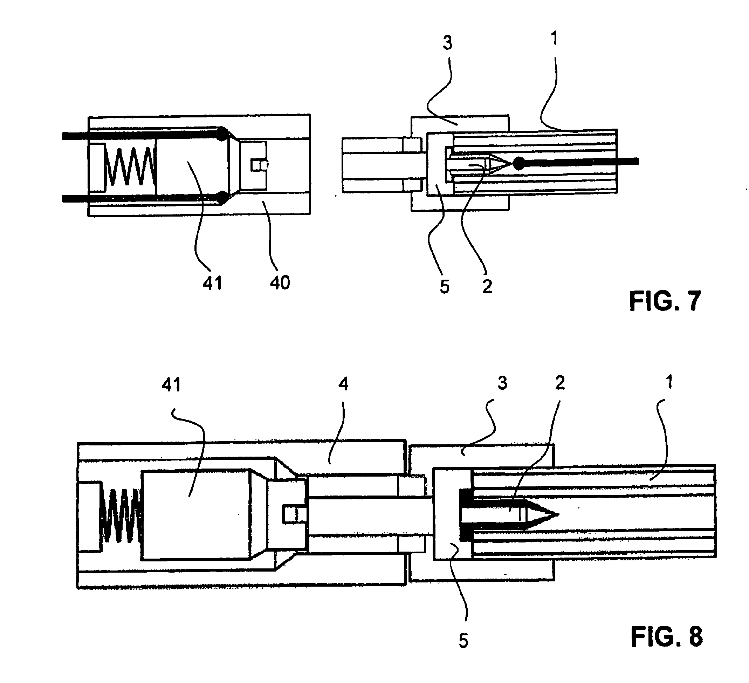

In Figur 6 ist mit dicken Linien die kommunizierende Verbindung in den einzelnen Kanälen und Leitungen dargestellt. Innerhalb der Drainageleitung 10 endet diese dicke Linie in einem Punkt. Dies soll darstellen, dass das zweite Ventil 2 geschlossen ist und somit diese Drainageleitung 10 an diesem Ende geschlossen ist. Dasselbe gilt für den Kanal im ersten Kupplungsteil 4. Hier ist das erste Ventil 41 geschlossen, so dass auch hier kein durchgehender Kanal vorhanden ist. Dieselbe Situation ist nochmals in Figur 7 dargestellt.

In Figur 8 sind die Kupplungsteile 3, 4 miteinander verbunden und das erste Ventil 41 gibt den Kanal frei. Das zweite Ventil 2 ist aber immer noch geschlossen.

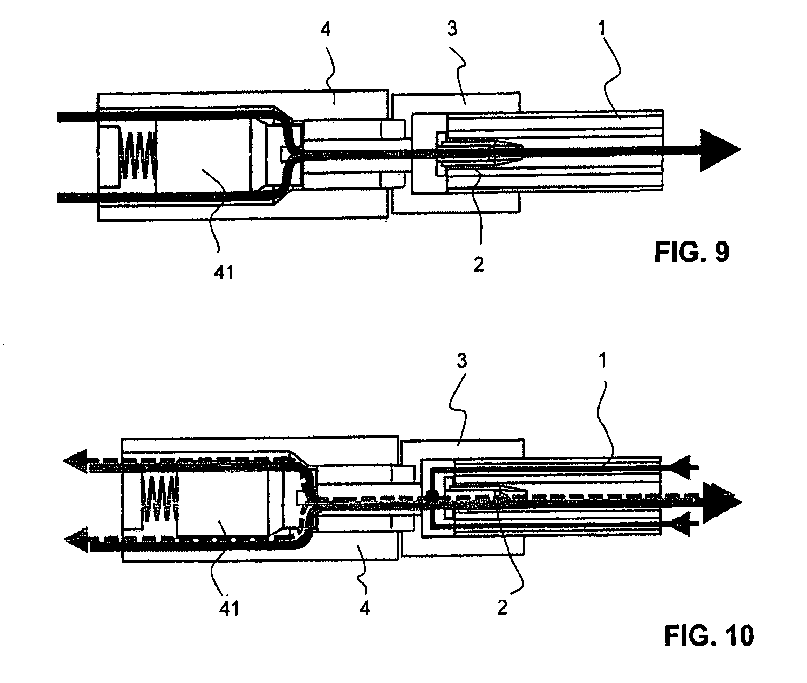

In Figur 9 stellt der dicke Pfeil den Fluss der abgesaugten Körperflüssigkeit dar. Dies ist ein Drainagebetrieb mit konstant angelegtem Unterdruck. Nun ist nicht nur das erste Ventil 41, sondern auch das zweite Ventil 2 geöffnet und der Kanal verläuft durchgehend durch die Kupplungseinheit vom Patienten zum Sekretsammelbehälter 61.

Figur 10 zeigt einen Betrieb mit intermittierend angelegtem Vakuum bzw. Unterdruck. Die Sequenzen können beispielsweise aus einer Saugphase von circa 6 Minuten und einer Pause unter Atmosphärendruck von circa 3 Minuten liegen. Während der Pause wird vorzugsweise nicht nur über die Zusatzleitungen 11 entlüftet, sondern es ergibt sich dadurch auch eine Spülung der Drainageleitung 10. Das zweite Ventil 2 ist offen.

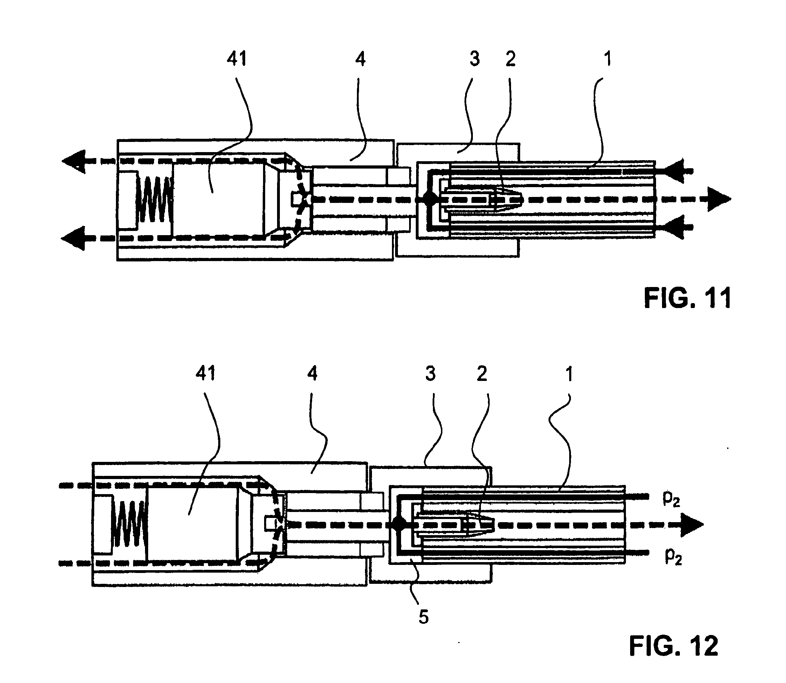

In Figur 11 ist eine Spülung bei konstant angelegtem Unterdruck dargestellt. Das zweite Ventil 2 ist offen und das Vakuum auf der Patientenseite, also am freien Ende des ersten Kupplungsteils 4, wird wie beim Betrieb in Figur 10 reduziert.

Figur 12 zeigt nun eine Druckmessung. Diese ist im Betrieb gemäss allen Figuren 9 bis 11 möglich.

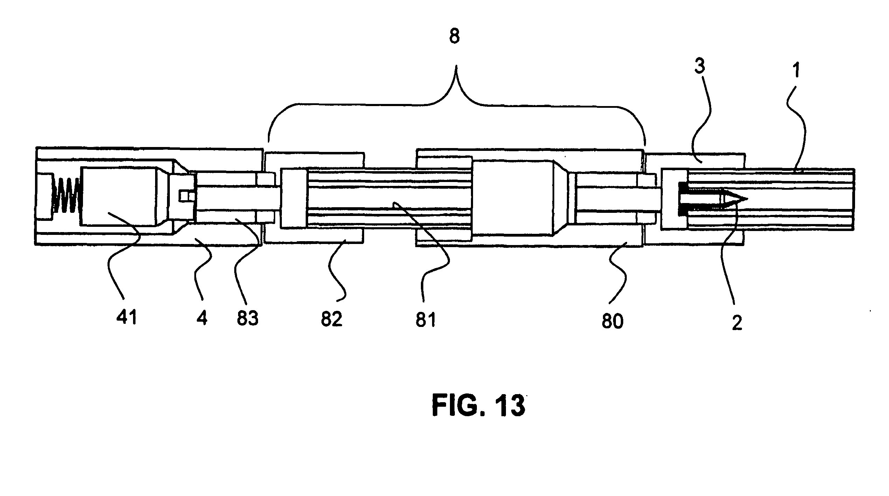

In Figur 13 ist eine Ausfuhrungsform dargestellt, in welcher das erste und das zweite Kupplungsteil 3, 4 nicht unmittelbar miteinander verbunden sind. Die zwei Kupplungsteile 3, 4 sind dabei gleich ausgestaltet wie im oberen Beispiel beschrieben. Das heisst, sie lassen sich auch unmittelbar ineinander stecken.

In Figur 8 ist ein Überbrückungsteil 8 vorhanden mit einem ersten Ende und einem zweiten Ende, wobei das erste Ende mit dem ersten Kupplungsteil 4 und das zweite Ende mit dem zweiten Kupplungsteil 3 verbindbar ist. Das erste Ende weist hierfür einen Grundkörper 82 auf, welcher vorzugsweise gleich ausgestaltet ist wie der oben beschriebene Grundkörper 36 des zweiten Kupplungsteils 3. Er ist zumindest funktionell gleich ausgestaltet. Im Grundkörper 36 ist wiederum ein Einsteckstutzen 83 angeformt, welcher vorzugsweise gleich ausgestaltet ist wie der Einsteckstutzen 33 des oben beschriebenen zweiten Kupplungsteils 3.

Das zweite Ende bildet das genaue oder zumindest funktionelle Ebenbild des entsprechenden Teils des ersten Kupplungsteils 4. Es weist einen Anschlussstutzen 80 auf, welcher dem Anschlussstutzen 400 des ersten Kupplungsteils 4 entspricht.

Der Mittelbereich 81 zwischen diesen zwei Enden des Überbrückungsteils 8 kann beliebig gestaltet sein.

Die erfindungsgemässe Drainageschlauchvorrichtung und die erfindungsgemässe Kupplungseinheit sind einfach aufgebaut und erlauben trotzdem viele Funktionen, insbesondere verhindern sie ein Nachtropfen bei Lösen der Verbindung und ermöglichen eine patientennahe Druckmessung.

**BEZUGSZEICHENLISTE**

| | | | |
|---|---|---|---|
| 1 | Drainageschlauch | 413 | zweiter Ventilkörper |
| 10 | Drainageleitung | 415 | Schlitz |
| 11 | Zusatzleitung | 42 | Schlauchanschluss |
| | | 420 | Vorsprung |
| 2 | zweites Ventil | 421 | Stützstruktur |
| 20 | Flansch | 43 | Dichtbereich |
| 21 | Schnabel | 44 | Aussengewinde |
| | | 44' | Stufen |
| 3 | zweites Kupplungsteil | 45 | Flansch |
| 30 | Anschlussstutzen | | |
| 301 | Vorsprung | 5 | Zwischenraum |
| 31 | Einführöffnung | | |
| 32 | Seitenrippe | 6 | Drainageeinheit |
| 33 | Einsteckstutzen | 60 | Vakuumpumpe |
| 34 | Fenster | 601 | erster Drucksensor |
| 35 | Druckbereich | 602 | zweiter Drucksensor |
| 36 | Grundkörper | 603 | Pumpaggregat |
| 37 | Rippen | 604 | Rückschlagventil |
| 38 | Gitter (Krone, Schlitzring) | 61 | Sekretsammelbehälter |
| | | | |
| 4 | erstes Kupplungsteil | 8 | Überbrückungsteil |
| 40 | Grundkörper | 80 | Anschlussstutzen |
| 400 | Anschlussstutzen | 81 | Mittelteil |
| 401 | Verbindungsstutzen | 82 | Grundkörper |
| 41 | erstes Ventil | 83 | Einsteckstutzen |
| 410 | erster Ventilkörper | | |
| 412 | Rippe | P | Patient |
| 412' | Ausnehmung | | |

## Patentansprüche

1. Drainageschlauchvorrichtung zum Absaugen von Flüssigkeiten aus einem menschlichen oder tierischen Körper, aufweisend
eine Kupplungseinheit mit einem ersten und einem zweiten Kupplungsteil (3,4), welche miteinander fluiddicht verbindbar sind,
ein erstes Ventil (41), welches im ersten Kupplungsteil (4) angeordnet ist und einen Drainageschlauch (1) mit einem patientenseitigen Ende, welches im zweiten Kupplungsteil (3) gehalten ist,
wobei der Drainageschlauch (1) mehrlumig ausgebildet ist und eine Drainageleitung (10) und mindestens eine Zusatzleitung (11) aufweist,
wobei diese Drainageleitung (10) und mindestens eine der genannten Zusatzleitungen (11) an ihrem patientenseitigen Ende innerhalb des zweiten Kupplungsteils (3) in fluid-kommunizierender Verbindung miteinander stehen, und
wobei die Drainageschlauchvorrichtung ferner ein zweites Ventil (2) aufweist, welches im zweiten Kupplungsteil (3) angeordnet ist und welches die Drainageleitung (10) öffnet und verschliesst.

2. Drainageschlauchvorrichtung nach Anspruch 1, wobei das erste Ventil (41) eine Strömung in zwei entgegen gesetzten Richtungen zulässt und verhindert.

3. Drainageschlaucheinheit nach einem der Ansprüche 1 oder 2, wobei im zweiten Kupplungsteil (3) am patientenseitigen Ende des Drainageschlauchs (1) ein Hohlraum (5) vorhanden ist, welcher die kommunizierende Verbindung zwischen der Drainageleitung (10) und der mindestens einen Zusatzleitung (11) erstellt und welcher eine Druckmessung, insbesondere eine Druckdifferenzmessung zwischen der mindestens einen Zusatzleitung (11) und der Drainageleitung (10), ermöglicht.

4. Drainageschlaucheinheit nach einem der Ansprüche 1 bis 3, wobei das zweite Ventil (2) auf das patientenseitige Ende des Drainageschlauchs (1) aufgesteckt ist.

5. Drainageschlaucheinheit nach einem der Ansprüche 1 bis 4, wobei das zweite Ventil (2) in die Drainageleitung (10) eingesteckt ist.

6. Drainageschlauchvorrichtung nach einem der Ansprüche 1 bis 5, wobei das zweite Ventil (2) zwischen einem Anschlag (38) des zweiten Kupplungsteils (3) und dem patientenseitigen Ende des Drainageschlauchs (1) in einem Presssitz gehalten ist.

7. Drainageschlauchvorrichtung nach einem der Ansprüche 1 bis 6, wobei das zweite Ventil (2) ein Einwegventil, vorzugsweise ein Entenschnabelventil, ist.

8. Drainageschlauchvorrichtung nach einem der Ansprüche 1 bis 7, wobei die zwei Kupplungsteile (3,4) lösbar miteinander verbindbar sind, vorzugsweise über eine Schnappverbindung.

9. Drainageschlauchvorrichtung nach einem der Ansprüche 1 bis 8, wobei ein Überbrückungsteil vorhanden ist mit einer ersten und einer zweiten Endkomponente und wobei die erste Endkomponente mit dem ersten Kupplungsteil und die zweite Endkomponente mit dem zweiten Kupplungsteil fluiddicht verbindbar sind.

10. Drainageschlauchvorrichtung nach einem der Ansprüche 1 bis 9, wobei das erste Ventil (41) einen deformierbaren Kolben (410) aufweist, welcher im ersten Kupplungsteil (4) gehalten ist und dessen Deformierung bei Verbindung des ersten Kupplungsteils (4) mit dem zweiten Kupplungsteil (3) respektive, falls vorhanden, mit dem Überbrückungsteil erfolgt.

11. Drainageschlauchvorrichtung nach einem der Ansprüche 1 bis 10, wobei die Kupplungseinheit (3,4) sensorlos ausgebildet ist.

12. Drainageschlauchvorrichtung nach einem der Ansprüche 1 bis 11, wobei die Drainageleitung (10) zentral im Drainageschlauch (1) angeordnet ist und die mindestens eine Zusatzleitung (11) peripher verläuft.

13. Drainageschlauchvorrichtung nach Anspruch 11, wobei mindestens drei, vorzugsweise genau drei Zusatzleitungen (11) vorhanden sind, welche gleichmässig über den Umfang des Drainageschlauchs (1) verteilt peripher angeordnet sind.

14. Kupplungseinheit zur Verwendung in einer Drainageschlauchvorrichtung gemäss einem der Ansprüche 1 bis 13, aufweisend
ein erstes und ein zweites Kupplungsteil (3, 4), welche miteinander fluiddicht verbindbar sind und
ein erstes Ventil (41), welches im ersten Kupplungsteil (4) angeordnet ist und wobei das zweite Kupplungsteil (3) eine Aufnahme zur Befestigung eines patientenseitigen Endes eines mehrlumigen Drainageschlauchs (1) aufweist, welcher eine Drainageleitung (10) und mindestens eine Zusatzleitung (11) aufweist, wobei die Aufnahme eine fluidkommunizierende Verbindung zwischen dieser Drainageleitung (10) und mindestens einer der genannten Zusatzleitungen (11) an ihrem patientenseitigen Ende innerhalb des zweiten Kupplungsteils (3) ermöglicht, und
wobei die Kupplungseinheit ferner ein zweites Ventil (2) aufweist, welches im zweiten Kupplungsteil (3) angeordnet ist und welches die Drainageleitung (10) öffnet und verschliesst.

## Claims

1. Drainage tube device for removing fluids by suction from a human or animal body, comprising
a coupling unit with first and second coupling parts (3, 4) that can be connected to each other in a leaktight manner,
a first valve (41) that is arranged in the first coupling part (4), and
a drainage tube (1) with an end that is directed towards the patient, which is held in the second coupling part (3),
wherein the drainage tube (1) has a multi-lumen design and a drainage conduit (10) and at least one auxiliary conduit (11),
wherein this drainage conduit (10) and at least one of said auxiliary conduits (11) are in fluidic communication with each other at their end directed towards the patient and within the second coupling part (3), and
wherein the drainage tube device further comprises a second valve (2), which is arranged in the second coupling part (3) and which opens and closes the drainage conduit (10).

2. Drainage tube device according to Claim 1, wherein the first valve (41) is a valve which allows or prevents a flow stream in two opposite directions.

3. Drainage tube unit according to one of Claims 1 and 2, wherein a hollow space (5) is present in the second coupling part (3), at the end of the drainage tube (1) directed towards the patient, which hollow space (5) creates the communicating connection between the drainage conduit (10) and the at least one auxiliary conduit (11) and permits a pressure measurement, in particular a measurement of the pressure difference between the at least one auxiliary conduit (11) and the drainage conduit (10).

4. Drainage tube unit according to one of Claims 1 to 3, wherein the second valve (2) is fitted onto the end of the drainage tube (1) directed towards the patient.

5. Drainage tube unit according to one of Claims 1 to 4, wherein the second valve (2) is fitted into the drainage conduit (10).

6. Drainage tube device according to one of Claims 1 to 5, wherein the second valve (2) is held in an interference fit between an abutment (38) of the second coupling part (3) and the end of the drainage tube (1) directed towards the patient.

7. Drainage tube device according to one of Claims 1 to 6, wherein the second valve (2) is a one-way valve, preferably a duckbill valve.

8. Drainage tube device according to one of Claims 1 to 7, wherein the two coupling parts (3, 4) can be connected to each other releasably, preferably via a snap-fit connection.

9. Drainage tube device according to one of Claims 1 to 8, wherein a bridging part with a first end component and a second end component is present, and wherein the first end component can be connected to the first coupling part and the second end component to the second coupling part in a leaktight manner.

10. Drainage tube device according to one of Claims 1 to 9, wherein the first valve (41) has a deformable piston (410) which is held in the first coupling part (4) and whose deformation takes place upon connection of the first coupling part (4) to the second coupling part (3) or, if it is present, to the bridging part.

11. Drainage tube device according to one of Claims 1 to 10, wherein the coupling unit (3, 4) is designed without sensors.

12. Drainage tube device according to one of Claims 1 to 11, wherein the drainage conduit (10) is arranged centrally in the drainage tube (1), and the at least one auxiliary conduit (11) extends peripherally.

13. Drainage tube device according to Claim 11, wherein at least three, preferably exactly three, auxiliary conduits (11) are present and are distributed peripherally in a uniform manner about the circumference of the drainage tube (1).

14. Coupling unit for use in a drainage tube device according to one of Claims 1 to 13, comprising first and second coupling parts (3, 4) that can be connected to each other in a leaktight manner, and a first valve (41) that is arranged in the first coupling part (4), and
wherein the second coupling part (3) has a recess for securing an end of a multi-lumen drainage tube (1) directed towards the patient, said drainage tube (1) having a drainage conduit (10) and at least one auxiliary conduit (11), wherein the recess permits fluidic communication between this drainage conduit (10) and at least one of said auxiliary conduits (11) at their end directed towards the patient and within the second coupling part (3), and
wherein the coupling unit further comprises a second valve (2), which is arranged in the second coupling part (3) and which opens and closes the drainage conduit (10).

## Revendications

1. Dispositif à tuyau souple de drainage pour l'aspiration de liquides hors d'un corps humain ou animal, présentant:
une unité d'accouplement avec une première et une deuxième parties d'accouplement (3, 4), qui peuvent être assemblées l'une à l'autre de façon étanche au fluide,
une première soupape (41), qui est disposée dans la première partie d'accouplement (4), et
un tuyau souple de drainage (1) avec une extrémité côté patient, qui est maintenu dans la deuxième partie d'accouplement (3),
dans lequel le tuyau souple de drainage (1) est réalisé avec plusieurs lumières et présente un conduit de drainage (10) et au moins un conduit supplémentaire (11),
dans lequel ce conduit de drainage (10) et au moins un desdits conduits supplémentaires (11) sont en liaison de communication fluidique l'un avec l'autre à l'intérieur de la deuxième partie d'accouplement (3) à leur extrémité côté patient, et
dans lequel le dispositif à tuyau souple de drainage présente en outre une deuxième soupape (2), qui est disposée dans la deuxième partie d'accouplement (3) et qui ouvre et ferme le conduit de drainage (10).

2. Dispositif à tuyau souple de drainage selon la revendication 1, dans lequel la première soupape (41) permet et empêche un écoulement dans deux directions opposées.

3. Unité à tuyau souple de drainage selon une des revendications 1 ou 2, dans laquelle il se trouve dans la deuxième partie d'accouplement (3), à l'extrémité côté patient du tuyau souple de drainage (1), une cavité (5) qui établit la liaison de communication entre le conduit de drainage (10) et ledit au moins un conduit supplémentaire (11), et qui permet une mesure de pression, en particulier une mesure de différence de pression entre ledit au moins un conduit supplémentaire (11) et le conduit de drainage (10).

4. Unité à tuyau souple de drainage selon l'une quelconque des revendications 1 à 3, dans laquelle la deuxième soupape (2) est emmanchée sur l'extrémité côté patient du tuyau souple de drainage (1).

5. Unité à tuyau souple de drainage selon l'une quelconque des revendications 1 à 4, dans laquelle la deuxième soupape (2) est engagée dans le conduit de drainage (10).

6. Dispositif à tuyau souple de drainage selon l'une quelconque des revendications 1 à 5, dans lequel la deuxième soupape (2) est maintenue en ajustement serré entre une butée (38) de la deuxième partie d'accouplement (3) et l'extrémité côté patient du tuyau souple de drainage (1).

7. Dispositif à tuyau souple de drainage selon l'une quelconque des revendications 1 à 6, dans lequel la deuxième soupape (2) est une soupape à une voie, de préférence une soupape en bec de canard.

8. Dispositif à tuyau souple de drainage selon l'une quelconque des revendications 1 à 7, dans lequel les deux parties d'accouplement (3, 4) peuvent être assemblées l'une à l'autre de façon séparable, de préférence par un assemblage à déclic.

9. Dispositif à tuyau souple de drainage selon l'une quelconque des revendications 1 à 8, dans lequel il se trouve une partie de pontage avec un premier et un deuxième composants d'extrémité et dans lequel le premier composant d'extrémité peut être assemblé de façon étanche au fluide à la première partie d'accouplement et le deuxième composant d'extrémité peut être assemblé de façon étanche au fluide à la deuxième partie d'accouplement.

10. Dispositif à tuyau souple de drainage selon l'une quelconque des revendications 1 à 9, dans lequel la première soupape (41) présente un piston déformable (410), qui est maintenu dans la première partie d'accouplement (4) et dont la déformation est produite lors de l'assemblage de la première partie d'accouplement (4) avec la deuxième partie d'accouplement (3), respectivement, si elle est présente, avec la partie de pontage.

11. Dispositif à tuyau souple de drainage selon l'une quelconque des revendications 1 à 10, dans lequel l'unité d'accouplement (3, 4) est réalisée sans capteur.

12. Dispositif à tuyau souple de drainage selon l'une quelconque des revendications 1 à 11, dans lequel le conduit de drainage (10) est disposé au centre dans le tuyau souple de drainage (1) et ledit au moins un conduit supplémentaire (11) se trouve en périphérie.

13. Dispositif à tuyau souple de drainage selon la revendication 11, dans lequel il se trouve au moins trois, de préférence exactement trois, conduits supplémentaires (11), qui sont disposés en périphérie de façon uniformément répartie sur la périphérie du tuyau souple de drainage (1).

14. Unité d'accouplement à utiliser dans un dispositif à tuyau souple de drainage selon l'une quelconque des revendications 1 à 13, présentant
une première et une deuxième parties d'accouplement (3, 4), qui peuvent être assemblées l'une à l'autre de façon étanche au fluide, et
une première soupape (41), qui est disposée dans la première partie d'accouplement (4), et
dans laquelle la deuxième partie d'accouplement (3) présente un logement pour la fixation d'une extrémité côté patient d'un tuyau souple de drainage (1) présentant plusieurs lumières, qui présente un conduit de drainage (10) et au moins un conduit supplémentaire (11), dans laquelle le logement permet une liaison de communication fluidique entre ce conduit de drainage (10) et au moins un desdits conduits supplémentaires (11) à leur extrémité côté patient à l'intérieur de la deuxième partie d'accouplement (3), et
dans laquelle l'unité d'accouplement présente en outre une deuxième soupape (2), qui est disposée dans la deuxième partie d'accouplement (3) et qui ouvre et ferme le conduit de drainage (10).
